# EUROPEAN PATENT APPLICATION

(11) **EP 1 632 564 A1**
(43) Date of publication of application: **08.03.2006**
(21) Application number: 04021065.0
(22) Date of filing: 03.09.2004
(51) Int. Cl.: C12N 7/04, C07K 14/165, A61K 39/215

(54) **Vaccine against severe accute respiratory syndrome causing coronavirus (SARS-CoV)**

(71) Applicant: CONSEJO SUPERIOR DE INVESTIGACIONES CIENTIFICAS, 28006 Madrid (ES)
(72) Inventor: Enjuanes Sanches, Luis, 28109 Madrid (ES); Almazan, Fernando, Dr., 28021 Madrid (ES)
(74) Representative: UEXKÜLL & STOLBERG

(57) **Abstract**

The present invention is directed to vaccines comprising a virus particle comprising a nucleic acid and one or several coat proteins, wherein
(a) the nucleic acid encodes SARS-CoV replicase, SARS-CoV N protein and at least one further SARS-CoV protein; and
(b) the one or several coat proteins are SARS-CoV coat proteins or proteins with a homology of at least 60% to SARS-CoV coat proteins necessary to allow the virus particle to infect human or animal cells,
wherein the nucleic acid is replication competent but not infectious.

## Description

The present invention is directed to virus particles, wherein these virus particles comprise a nucleic acid and one or several coat proteins. The nucleic acid within the virus particle encodes SARS-CoV replicase, SARS-CoV N protein and at least one further SARS-CoV protein. The one or several coat proteins of the virus particle are SARS-CoV coat proteins necessary to allow the virus particle to infect human or animal cells. The virus particles are further replication competent but not infectious.

### TECHNICAL BACKRGOUND

Therapy approaches that involve the insertion of a functional gene into a cell to achieve a therapeutic effect are also referred to as gene therapy approaches, as the gene serves as a drug. Gene therapy is a technique primarily for correcting defective genes responsible for disease development.

A carrier molecule also referred to as a vector is used to deliver the therapeutic gene to the patient's target cells. Currently, the most common vector is a virus that has been genetically altered to carry human or animal genes. Viruses have evolved a way of encapsulating and delivering their genes to human or animal cells in a pathogenic manner. Scientists have taken advantage of this capability and manipulate the virus genome to remove disease-causing genes and insert therapeutic genes.

Target cells such as the patient's liver or lung cells are infected with the viral vector. The vector then unloads its genetic material containing the therapeutic gene into the target cell. The generation of a functional protein product from the therapeutic gene restores the target cell to a normal state.

In an alternative approach, these viral vectors were used for expressing heterologous genes that cause an immunogenic response in the subject receiving the vector and thus immunize that subject. In that case the viral vector serves as a vaccine.

Since the etiologic pathogen causing SARS was identified as a new coronavirus (Drosten et al., 2003; Holmes and Enjuanes, 2003; Marra et al., 2003; Rota et al., 2003), the study of coronavirus molecular biology was given a very high priority in order to develop effective strategies to prevent and control coronavirus infections.

Coronaviruses are ssRNA(+) viruses which have the largest genome so far found in RNA viruses with a length between 25 and 31 kilobases (kb; see Siddell S.G. 1995, The Coronaviridae). When a coronavirus infects a cell, the genomic RNA (gRNA) replicates in the cytoplasm and a set of subgenomic RNAs (sgRNA) of positive and negative polarity is produced (Sethna et al., 1989; Sawicki & Sawicki, 1990; and Van der Most and Spaan, The Coronaviridae).

Due to the fact that the coronaviruses replicate in the cytoplasm, use of coronaviruses as a vector for gene therapy and vaccination has been suggested. Specifically, defective interfering (DI) genomes of coronaviruses were produced. These DI genomes are deletion mutants which require the presence of a complementing or helper virus for replication and/or transcription (see Chang et al., 1994; WO97/34008; Spanish patent application P9600620; Izeta et al., 1999; and Sánchez et al., 1999).

A respective system was used in the art to generate immune responses in an animal which received a composition containing a DI genome which amongst others contained sequences encoding a heterologous reporter gene or a gene derived from a different infectious agent (porcine reproductive and respiratory disease virus, PRRSV; see Alonso et al., 2002a, 2002b).

The construction of coronavirus full-length cDNA clones (Almazán et al., 2000; Casais et al., 2001; Thiel et al., 2001; Yount et al., 2000; Yount et al., 2002; Yount et al., 2003) provided the opportunity for the genetic manipulation of coronavirus genomes to study fundamental viral processes and to develop expression vectors.

Although SARS-CoV emerged only in 2002, genome sequences of SARS-CoV isolates have been published recently and provide important information on the organization, phylogeny and variability of the SARS-CoV genome (Marra et al., 2003; Rota et al., 2003). About two-thirds of the 29.7-kb Urbani strain genome encode the replicase gene that comprises open reading frames Rep 1a and Rep 1b, the latter one being expressed by ribosomal frameshifting (Thiel et al., 2003). Translation of both ORFs results in the synthesis of two large polyproteins that are processed by viral proteinases to yield the replicase-transcriptase complex (Ziebuhr et al., 2000).

The 3' one-third of the genome includes the genes encoding the structural and non-structural proteins, in the order 5'-S-3a-3b-E-M-6-7a-7b-8a-8b-9b-N-3'. These proteins are expressed by a discontinuous transcription process that most probably takes place during the synthesis of the negative strand, leading to the generation of a 3' coterminal nested set of subgenomic mRNAs, each of which has at its 5' end a capped leader sequence derived from the 5' end of the genome (Sawicki and Sawicki, 1998; Zúñiga et al., 2004). Synthesis of subgenomic negative sense RNA species is regulated by the transcription-regulating sequences (TRSs), that include a highly conserved core sequence that is found preceding each gene and at the 3' end of the leader sequence (Thiel et al., 2003).

SARS-CoV first broke out in humans in late 2002 and spread within a few months from its origin in Guangdong (China) to more than 30 countries. The rapid transmission and high mortality rate made SARS a global threat for which no efficacious therapy is available. The problem underlying the present invention thus resides in providing vaccines for protection against SARS with good safety and immunogenicity.

### SUMMARY OF THE INVENTION

The present invention provides vaccines comprising a virus particle, wherein the virus particle comprises a nucleic acid and one or several coat proteins. The nucleic acids encode SARS-CoV replicase, SARS-CoV N protein and at least one further SARS-CoV protein and are replication competent but not infectious. The coat proteins are SARS-CoV coat proteins necessary to allow the virus particle to infect human or animal cells. A virus particle comprising all coat proteins of a virus and a replication competent nucleic acid encoding a not all proteins necessary for infection is also referred to as a pseudovirus in the art. The vaccine can be administered to a human or an animal to reduce or eliminate the symptoms of a subsequent SARS virus infection.

According to another aspect of the present invention the further SARS-CoV proteins encoded by the nucleic acid are proteins capable of associating into SARS-CoV virus like particles.

The vaccines of the present invention may be administered to a mammal to produce an immune response that reduces or eliminates the disease symptoms caused by infection with the SARS virus. The vaccines of the present invention may further comprise pharmaceutically acceptable carrier and/or adjuvants.

### DETAILED DESCRIPTION OF THE INVENTION

The present invention is directed to a vaccine comprising virus particles comprising a nucleic acid and one or several coat proteins, wherein:
(a) the nucleic acid encodes SARS-CoV replicase, SARS-CoV N protein and at least one further SARS-CoV protein; and
(b) the one or several coat proteins are SARS-CoV coat proteins or proteins with a homology of at least 60% to wild type SARS-CoV coat proteins necessary to allow the virus particle to infect human or animal cells,

wherein the nucleic acid is replication competent but not infectious.

The present inventors have surprisingly found that a vaccine comprising a virus particle as defined above is safe and can initiate an immune response against SARS viruses. The vaccine comprises a virus particle that contains all elements necessary to infect human or animal cells. Upon infection of a cell, the virus will be able to replicate but will no longer be able to produce an infective virus particle, as the nucleic acid of the virus particle comprises less than all genes necessary to produce an infectious virus particle. Replication of the viral nucleic acid is obtained by the viral replicase, however, replication is significantly increased by the additional presence of the viral N protein. The nucleic acid of the virus particle further encodes at least one SARS protein that will be expressed. The high copy number of the nucleic acid obtained due to the presence of the replicase and N protein will increase expression of the at least one further SARS protein. Expression of the at least one further SARS protein is suitable to generate an immune response in a mammal vaccinated with the vaccines of the present invention.

"Replication competent" nucleic acid as used herein means that the nucleic acid replicates itself autonomously when introduced in a cell. The nucleic acid thus comprises sequences encoding all proteins necessary for replication.

SARS-CoV coat proteins are proteins, such as the SARS-CoV E, M or S (spike) protein or proteins. The proteins may have a sequence as observed in a SARS isolate or may be derived therefrom by methods known in the art. Using methods of recombinant expression it is for example possible to modify protein sequences by addition, deletion or substitution of one or several amino acids. The present invention thus covers viruses comprising or encoding coat proteins with a sequence homology of at least 60%, preferably at least 75% and most preferably at least 95% to the wild type SARS-CoV proteins.

For the purposes of the present application sequence homology is determined using the Clustal computer program available from the European Bioinformatics Institute (EBI), unless otherwise stated.

In accordance with the present invention vaccines are provided, which are preferably capable of inducing both a systemic immune response and a mucosal immune response against SARS-CoV.

In one embodiment of the present invention the SARS-CoV replicase is encoded by SEQ ID NO:1 (Rep1a) and SEQ ID NO:2 (Rep1b) or a nucleic acid having a homology of at least 60%, preferably at least 75% and most preferably at least 95% to SEQ ID NOs:1 or 2. SEQ ID NOs:1 and 2 contain a partial overlap at the beginning of SEQ ID NO:2, as the Rep1b open reading frame is translated after a ribosomal framshift of -1. The replicase gene may thus be encoded by a single nucleic acid, wherein the overlapping sequence is present only once or by two seperate sequences as shown in SEQ ID Nos: 1 and 2.

In another embodiment the SARS-CoV N protein is encoded by SEQ ID NO:3 or a nucleic acid having a homology of at least 60%, preferably at least 75% and most preferably at least 95% to SEQ ID NO:3.

In a preferred embodiment of the present invention the nucleic acid further encodes the SARS-CoV spike protein. In a more preferred embodiment the SARS-CoV spike protein is encoded by SEQ ID NO:4 or a nucleic acid having a homology of at least 60%, preferably at least 75% and most preferably at least 95% to SEQ ID NO:4.

In a most preferred embodiment the nucleic acid does not encode any other protein except for SARS-CoV replicase, SARS-CoV N protein and SARS-CoV spike protein.

Similar to other coronaviruses, the spike protein of the virus interacts with a cellular receptor and mediates membrane fusion to allow viral entry into susceptible target cells. Accordingly, the spike protein plays an important role in virus infection cycle and is the primary target of neutralizing antibodies.

In another preferred embodiment of the present invention the nucleic acid further encodes the SARS-CoV M protein. In a more preferred embodiment the SARS-CoV M protein is encoded by SEQ ID NO:5 or a nucleic acid having a homology of at least 60%, preferably at least 75% and most preferably at least 95% to SEQ ID NO:5.

In the most preferred embodiment the nucleic acid does not encode any other protein except for SARS-CoV replicase, SARS-CoV N protein and SARS-CoV M protein.

In yet another preferred embodiment of the present invention the nucleic acid further encodes the SARS-CoV E protein. In a more preferred embodiment the SARS-CoV E protein is encoded by SEQ ID NO:6 or a nucleic acid having a homology of at least 60%, preferably at least 75% and most preferably at least 95% to SEQ ID NO:6.

In a most preferred embodiment the nucleic acid does not encode any other protein except for SARS-CoV replicase, SARS-CoV N protein and SARS-CoV E protein.

The M and E protein are structural proteins which are part of the lipid envelope of coronaviruses.

The invention is also directed to vaccines, wherein the nucleic acid further encodes SARS-CoV M protein and SARS-CoV E protein, or SARS-CoV M protein and SARS-CoV spike protein, or SARS-CoV E protein and SARS-CoV spike protein or SARS-CoV M protein, SARS-CoV E protein and SARS-CoV spike protein. These combinations may also be encoded by nucleic acid sequences having at least 60%, preferably at least 75% and most preferably at least 95% homology to the SARS-CoV wild type nucleic acid sequences.

In another preferred embodiment of the invention the nucleic acid further encodes SARS-CoV proteins capable of associating into SARS-CoV virus like particles.

In the present application the term "virus-like particles" (or VLPs) is used to refer to an association of several proteins of one virus that resembles the virus. The VLPs do not contain any nucleic acid. The proteins may be covalently coupled or otherwise linked and may have the same or a different sequence. Preferably the particle comprises an association of several different viral coat proteins.

While the nucleic acids may encode any SARS-CoV protein capable of associating into a VLP, in a preferred embodiment of the present invention they encode SARS-CoV proteins M, SARS-CoV E protein and SARS-CoV spike protein or proteins with a homology of at least 60% , preferably at least 75% or most preferably at least 95% to these proteins, wherein SARS-CoV M, E and spike proteins form virus-like particles. These VLPs will initiate immune responses closely mimicking the immune response caused by the wild-type virus particle.

In another embodiment of the invention the nucleic acid further encodes co-stimulating molecules, for example CD80 or CD86 or immunostimulating cytokines, for example TNF-alpha, IFN-gamma, granulocyte/macrophage colony-stimulating factor, interleukin-2 (IL-2), IL-12 or IL-18.

The vaccine of the present invention may be administered to a mammal to obtain an immune response that reduces or eliminates the disease symptoms caused by infection with the SARS virus. The vaccine is preferably used for vaccinating a mammal, especially a human.

The vaccine may be administered in accordance with methods routinely used in the art. Specifically vaccine may be administered by intramuscular, intravenous, subcutaneous or intranasal administration.

The vaccines may also comprise pharmaceutically acceptable carriers, excipients and/or adjuvants.

Adjuvants and carriers suitable for administering genetic vaccines and immunogens via the mucosal route are known in the art. Conventional carriers and adjuvants are for example reviewed in Kiyono et al. 1996. The addition of chemokines that are used to modulate immune responses are also encompassed by the present invention. Respective compounds and their medical use has been reviewed in Toka et al. 2004. It is specifically advantagous to use one of granulocyte/macrophage colony-stimulating factor, interleukin-2 (IL-2), IL-12, IL-18. Combinatorial approaches utilizing several cytokines and chemokines might also be applied. In addition, as more is discovered regarding the requirements for memory development of T cells, boosters involving key cytokines such as IL-15 and IL-23 may prove beneficial to long-term maintenance of the memory pool.

The present invention is also directed to nucleic acids, wherein these nucleic acids are SARS-CoV replicons. The term "Replicon" is used in the present application to refer to a nucleic acid capable of autonomous replication, the nucleic acid thus encodes all proteins that are needed to replicate the nucleic acid upon inserting the nucleic acid into a host cell. The nucleic acid may further comprise other SARS sequences, but is preferably non-infectious, i.e. does not encode proteins that are capable of associating with the nucleic acid to form a virus particle that is capable of infecting other cells.

In one embodiment the replicon encodes SARS-CoV replicase and SARS-CoV N protein. In a preferred embodiment the replicon encodes at least one further SARS-CoV protein, preferably the SARS-CoV spike protein. The invention also comprises replicons, wherein the nucleic acids encoding SARS-CoV proteins have a homology of at least 60%, preferably at least 75% and most preferably at least 95% to the SARS-CoV wild-type nucleic acids.

Methods to prepare the nucleic acids and the virus particles of the present invention may comprise the preparation of a SARS-CoV derived replicon in a bacterial artificial chromosome (BAC). The SARS sequences may be based on the Urbani strain genome (Genebank, accession number AY278741). The virus particles in the vaccines of the present invention are preferably prepared starting from a SARS replicon.

A replicon can be generated using a set of overlapping cDNAs spanning the untranslated 5' and 3' ends of the genome, the entire replicase gene, and the nucleoprotein (N) gene.

The strategy for the construction of the SARS-CoV replicon as a bacterial artificial chromosome (BAC) can be summarized as follows:
(i) Identification of appropriate restriction sites in the viral genome to be used in the engineering of the replicon.
(ii) Generation of an intermediate plasmid as the basis for construction of the SARS-CoV replicon.
(iii) Generation of overlapping SARS-CoV cDNA subclones by RT-PCR and replicon assembly using the restriction sites selected.

The construction of a SARS-CoV replicon cDNA is illustrated with further details in Example 1.

Methods for preparing the virus particles used in the vaccines of the present invention may use a replicon as described above. According to a preferred aspect of the present invention these methods are based on the use of the plasmid pBAC-SARS-CoV-REP. Bacteria containing this plasmid were deposited on September 1, 2004 with the Colección Espanola de Cultivos (Tipo, CECT in Valencia, Spain). The deposit was given the provisional accession number 7020.

The plasmid pBAC-SARS-CoV-REP comprises sequences of a replication competent, non-infectious SARS genome encoding the SARS-CoV replicase and the SARS-CoV N protein. This vector provides a stable, safe and easy to handle basis for producing the virus particles and the vaccines of the present invention. The virus particles and the vaccines may be produced by cloning the at least one further SARS gene into the replicon.

Genomic viral RNA may be used as a starting material to obtain the sequences encoding the further SARS proteins. A cDNA library is prepared from the virus RNAs according to methods known in the art. The full length genes can be reconstructed from the cDNA library. These genes may then be combined with transcriptional regulatory sequences (TRS) and/or translation regulatory sequences (such as internal ribosome entry sites, IRES) and can be inserted into a cloning site of the replicon.

The nucleic acid may then be used to transfect a helper cell line expressing the essential SARS proteins not encoded by the nucleic acid. Alternatively, a host cell may be transfected with the nucleic acid as described above and a helper virus or other nucleic acid expressing the essential SARS proteins. In this manner the nucleic acid encoding SARS-CoV replicase, SARS-CoV N protein and at least one further SARS-CoV protein will associate with the further essential SARS proteins to form the virus particle of the vaccines of the present invention.

The vaccines can be obtained by producing a virus particle as outlined above and formulating the same into a vaccine, for example by mixing the same with a carrier, adjuvants and/or excipient.

The testing of the vaccines preferably comprises a sequence of several steps. For example the immunogenicity of SARS-CoV VLPs may be tested by producing them in tissue culture of Vero cells. The antigens obtained can be partially purified by conventional procedures including centrifugation and it can be tested whether these induce SARS-CoV neutralizing antibodies in mice and rabbits.

Selected vaccines may further be tested for protection in animal systems, such as BalB/c mice, ferrets and macaques (using conventional methods as described for example in Enserink, 2003; Yang et al., 2004; ter Meulen et al., 2004; Martina et al., 2003; and Kuiken et al., 2004).

The animal model systems can be sequentially used for vaccine efficacy and preliminary safety testing. First, vaccine candidates can be tested in the mice model, then in the ferret SARS-CoV model (Martina et al., 2003). Further decisions can be based on protection against SARS-CoV challenge, the selected vaccine candidates may then be tested in macaques. To achieve this goal, a virus stock can be titrated in young adult cynomolgus macaques. In first experiments increasing logarithmic dilutions of this virus stock (10⁰, 10¹, 10², 10³, 10⁴, 10⁵ and 10⁶ TCID₅₀) should be used to infect macaques intra-tracheally (n= 2 per dilution). The clinical, virological, gross pathological and immunological (neutralizing antibodies, cytokine profiles in plasma and cells by mRNA analyses) data may be collected systematically from these SARS-CoV-infected animals, according to protocols that have been established previously (Kuiken et al., 2004).

### Brief description of the figures:

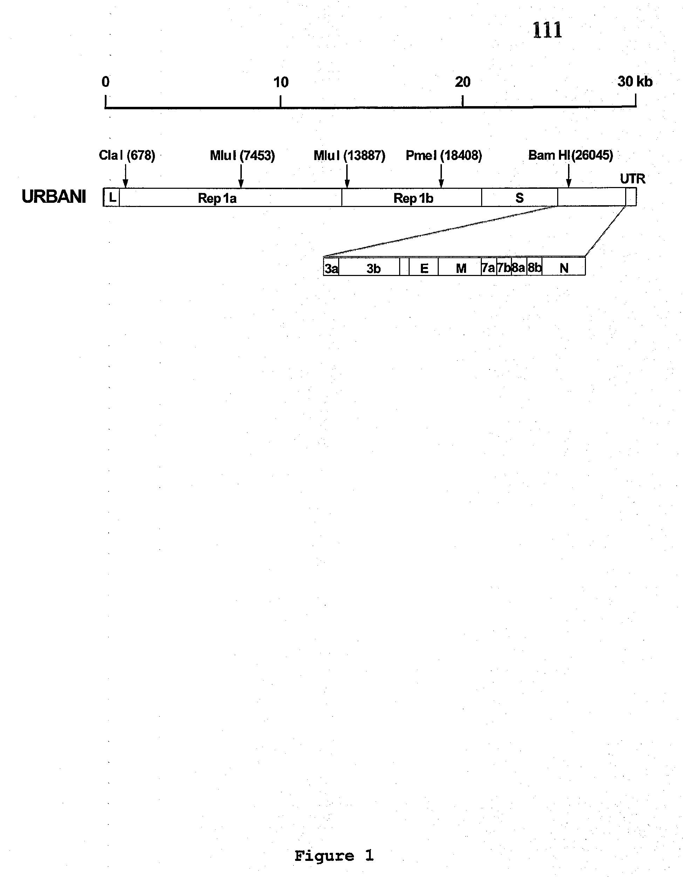

Figure 1 shows the genetic structure of SARS-CoV Urbani strain. Letters and number inside the boxes indicate the viral genes. L, leader sequence; UTR, untranslated region. Relevant restriction sites are indicated.

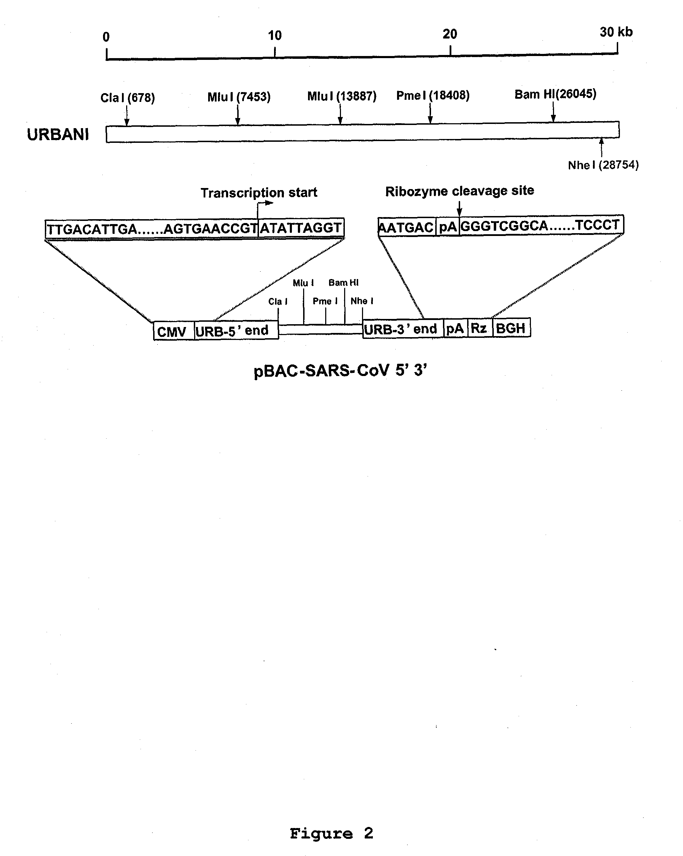

Figure 2 shows the construction of the intermediate plasmid pBAC-SARS-CoV 5' 3'. A PCR-fragment containing the 5' end 678 nt and the 3' end 973 nt of the genome of SARS-CoV Urbani strain, flanked at its left end by the cytomegalovirus (CMV) immediate-early promoter and at its right end by a poly (A) tail followed by the hepatitis delta virus ribozyme (Rz) and the bovine growth hormone (BGH) termination and polyadenylation sequences, is cloned in BAC. To facilitate the assembly of SARS-CoV replicon, a multicloning site containing the restriction sites *Cla* I, *Mlu* I, *Pme* I, and *Bam* HI, is cloned downstream of the 5' end viral sequence.

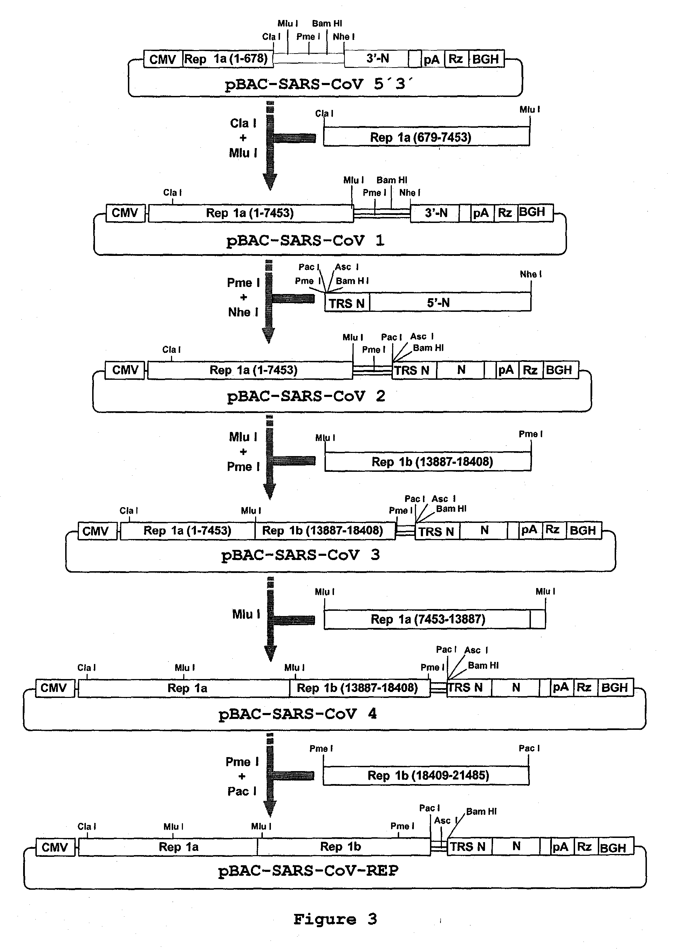

Figure 3 shows the generation of the SARS-CoV derived replicon pBAC-SARS-CoV-REP. The strategy for the construction of a SARS-CoV replicon is illustrated. SARS-CoV replicon is assembled by sequential cloning of subgenomic overlapping cDNA fragments generated by RT-PCR, into plasmid pBAC-SARS-CoV 5' 3'. The genetic map of SARS-CoV replicon is shown at the bottom. Relevant restriction sites used in the cloning step are indicated. Rep 1a, Rep 1b, and N indicate the viral genes. CMV, CMV immediate-early promoter; TRS N, natural TRS of N gene; pA, tail of 25 A residues; Rz, hepatitis delta virus ribozyme; BGH, bovine growth hormone termination and polyadenylation sequences.

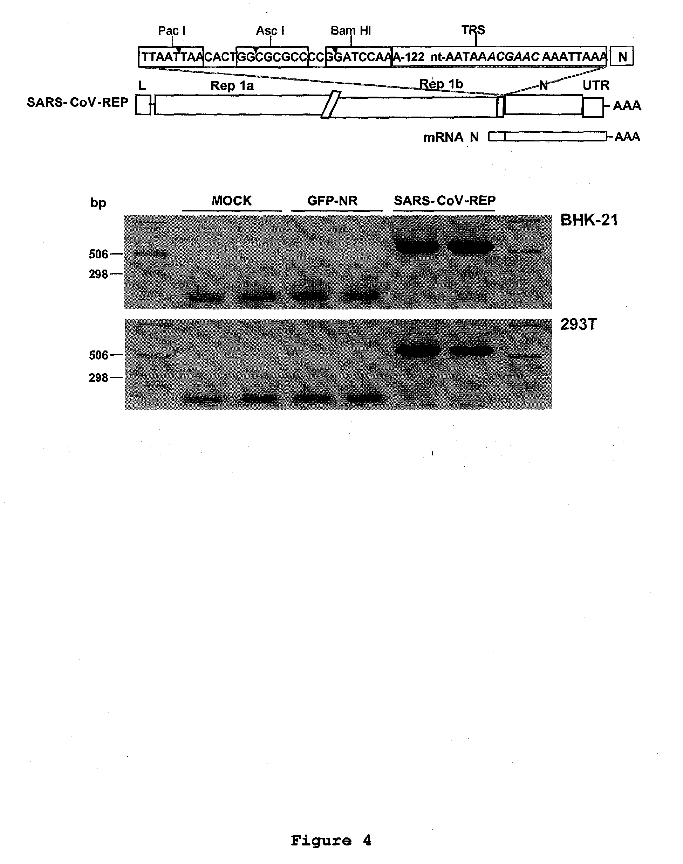

Figure 4 shows the functional analysis of SARS-CoV derived replicon. The genetic structure of the SARS-CoV replicon is illustrated at the top. The N gene TRS , the core sequence (italic letters), and the relevant restriction sites are indicated. L, leader sequence; UTR, untranslated region. BHK-21 and human 293T cells are mock transfected (MOCK) or transfected with SARS-CoV replicon (SARS-CoV-REP) or a non-replicative cDNA clone (GFP-NR) using Lipofectamine 2000. Total RNA is isolated at 24 hpt and analyzed by RT-PCR with specific oligonucleotides to detect gene N mRNA. Duplicate RT-PCR products amplified in parallel were resolved by electrophoresis in 1% agarose gels.

The following examples illustrate but do not limit the embodiments of the invention.

### EXAMPLES

The following cells, viruses, plasmids and bacteria strains were used:

**Cells and viruses.** Baby hamster kidney cells (BHK-21) and human 293T cells were purchased from the American Collection of Cell Cultures (ATCC). All cells were maintained in DMEM supplemented with 10% fetal bovine serum and antibiotics at 37°C in a CO₂ incubator. The genomic RNA of Urbani strain was obtained from the Center for Disease Control and Prevention (CDC) after signature of a material transfer agreement.

**Plasmids and bacteria strains**. Plasmid pBeloBAC11 (Wang et al., 1997) was kindly provided by H. Shizuya and M. Simon (California Institute of Technology, Pasadena, Ca). *Escherichia coli* DH10B strain was obtained from GIBCO/BRL. DH10B cells were transformed by electroporation at 25 µF, 2.5 kV, and 200 Ω with a Gene Pulser unit (Bio-Rad) according to the manufacturer's instructions. Plasmid DNA was isolated with the Qiagen (Chatsworth, CA) Large Construct Kit according to the manufacturer's specification.

### EXAMPLE 1

### Construction of a SARS-CoV replicon cDNA

A cDNA that contained the untranslated 5' and 3' end of the Urbani genome and the replicase and N genes, was cloned as a BAC under the control of a CMV promoter.

Additionally, a multicloning site containing unique restriction sites *Pac* I, *Asc* I, and *Bam* HI, was cloned downstream of the replicase gene to allow cloning of heterologous genes (Fig. 3). This approach use a two-step amplification system that couples replicon RNA transcription in the nucleus from the CMV promoter with a second amplification step in the cytoplasm driven by the viral polymerase. The plasmid pBAC-SARS-CoV-REP, encoding the SARS-CoV replicon, was stable for at least 180 generations during its propagation in DH10B cells, as determined by restriction endonuclease analysis.

In a first step the appropriate restriction sites in the viral genome that can be used in the engineering of the replicon were identified (Fig. 1).

In a second step the intermediate plasmid pBAC-SARS-CoV 5' 3' was generated as the basis to construct the SARS-CoV replicon (Fig. 2). This plasmid contained the first 678 nt of the 5'end of the Urbani strain genome under the control of the cytomegalovirus (CMV) immediate-early promoter and the last 973 nt of the genome followed by a 25-bp synthetic poly (A), the hepatitis delta virus ribozyme, and the bovine growth hormone termination and polyadenylation sequences to make an accurate 3' end. Additionally, a multicloning site containing the restriction sites *Cla* I, *Mlu* I, *Pme* I, and *Bam* HI, selected in the first step, was cloned in between the SARS-CoV sequences to allow the assembly of the SARS-CoV replicon.

In a third step the overlapping SARS-CoV cDNA subclones were generated by RT-PCR and the replicon was assembled using the restriction sites selected. Using the pBAC-SARS-CoV 5' 3' as starting point, a SARS-CoV replicon was engineered following the strategy described in Figure 3. The plasmid pBAC-SARS-CoV-REP contained the untranslated 5' and 3' ends of the Urbani genome, the replicase gene followed by a multicloning site containing unique restriction sites *Pac* I, Asc I, and *Bam* HI, to allow cloning and expression of heterologous genes, and the nucleoprotein (N) gene under the control of its natural TRS.

### EXAMPLE 2

### Analysis of cloned cDNA stability

The stability of the viral sequences cloned into pBeloBAC11 was analyzed by studying the restriction endonuclease pattern at different passages. Bacteria transformed with recombinant plasmid were grown in 10 ml of LB containing 12.5 *µ*g/ml chloramphenicol at 37°C. Cells from these primary cultures (considered passage 0) were propagated serially by diluting 10⁶-fold daily. Each passage was considered to represent about 20 generations.

### EXAMPLE 3

### Sequence analysis

DNA was sequenced using an automatic 373 DNA sequencer (Applied Biosystem) using fluorochrome labeled dideoxynucleotides and temperature resistant DNA polymerase (Perkin Elmer).

### EXAMPLE 4

### Transfection of SARS-CoV replicon cDNA

BHK-21 and 293T cells were grown to 95% confluence on 35-mm-diameter plates and transfected with 5 µg of the SARS-CoV replicon, using 6 µg of Lipofectamine 2000 (Invitrogen) according to the manufacturer's specifications.

### EXAMPLE 5

### RNA isolation and RT-PCR analysis

Total intracellular RNA was extracted at 24 h post-transfection (hpt) with the RNeasy Mini Kit (Qiagen) and used as template for RT-PCR analysis of gene N mRNA transcription. RT reactions were performed with Moloney murine leukaemia virus reverse transcriptase (Ambion) and the antisense primer URB-28630RS (5'-TGCTTCCCTCTGCGTAGAAGCC-3') (SEQ ID NO:7), complementary to nucleotides 510 to 531 of gene N. The cDNAs generated were amplified by PCR using the reverse primer URB-28630RS and the forward primer URB-29VS (5'-GCCAACCAACCTCGATCTCTTG-3') (SEQ ID NO:8), spanning nucleotides 29 to 50 of the Urbani leader sequence. For the quantitative analysis by real-time RT-PCR of gene N mRNA, the primers used for RT (URB-28163RS, 5'-TGGGTCCACCAAATGTAATGC-3' (SEQ ID NO:9), complementary to nucleotides 43 to 63 of gene N) and PCR (reverse primer URB-28163RS and the forward primer URB-27VS, 5' -AAGCCAACCAACCTCGATCTC-3' (SEQ ID NO:10), spanning nucleotides 27 to 47 of the Urbani leader sequence) were designed using the Primer Express software (Applied Biosystems). The SYBR Green PCR master mix was used in the PCR step following the manufacturer's specifications (Applied Biosystems).

### EXAMPLE 6

### Analysis of the SARS-CoV derived replicon

The SARS-CoV derived replicon was functional in several cell lines. Expression of gene N mRNA was used to study replicon activity by RT-PCR analysis. BHK-21 and human 293T cells were transfected with either SARS-CoV replicon or a non-replicative cDNA clone using Lipofectamine 2000. Total intracellular RNA was extracted at 24 hpt and used as template for RT-PCR analysis of gene N mRNA transcription using specific oligonucleotides. High levels of gene N mRNA were detected in both BHK-21 and 293T cells transfected with the SARS-CoV replicon (Fig. 4), showing that the replicon isactive in these cell lines. A quantitative analysis of gene N mRNA in transfected 293T and BHK-21 cells was performed by real-time RT-PCR using the primers described in Example 5. The SARS-CoV replicon activity was 8-fold higher in 293T cells than in BHK-21 cells. This activity increase could be explained by a higher replication level of SARS-CoV replicon in human 293T cells or simply because the transfection efficiency of 293T cells is twice higher than that of BHK-21 cells.

The role of the N protein in SARS-CoV replicon activity was analyzed. To achieve this objective, a SARS-CoV replicon lacking the N gene was constructed and the replicon activity compared with that of the replicon expressing the N gene. A basal activity of the replicon lacking N gene was detected, but replicon activity increased more than 100-fold when N protein was present. These data indicated that N protein plays an important role as an enhancer of coronavirus replicon activity.

Bacteria containing the plasmid pBAC-SARS-CoV-REP comprising sequences of a replication competent, non-infectious SARS genome encoding the SARS-CoV replicase and the SARS-CoV N protein were deposited on September 1, 2004 with the Colección Espanola de Cultivos (Tipo, CECT in Valencia, Spain). The deposit was given the provisional accession number 7020.

### Bibliography

Almazán, F., J. M. González, Z. Pénzes, A. Izeta, E. Calvo, J. Plana-Durán, and L. Enjuanes (2000). Engineering the largest RNA virus genome as an infectious bacterial artificial chromosome. Proc. Natl. Acad. Sci. USA 97:5516-5521.

Alonso, S., Izeta A., Sola I., and Enjuanes L. (2002a). Transcription regulatory sequences and mRNA expression levels in the coronavirus transmissible gastroenteritis virus. J. Virol. 76:1293-1308

Alonso, S., Sola, I., Teifke, J., Reimann, I., Izeta, A., Balach, M., Plana-Durán, J., Moormann, R. J. M., and Enjuanes, L. (2002b). In vitro and in vivo expression of foreign genes by transmissible gastroenteritis coronavirus-derived minigenomes. *J. Gen. Virol.* **83**, 567-579.

Casais, R., V. Thiel, S. G. Siddell, D. Cavanagh, and P. Britton (2001). Reverse genetics system for the avian coronavirus infectious bronchitis virus. J. virol. **75**:12359-12369.

Chang, K.-Y., and Tinoco, I. (1994). Characterization of a "kissing" hairpin complex derived from the human immunodeficiency virus genome. *Proc*. *Natl. Acad. Sci. USA* **91**, 8705-8709.

Drosten, C., S. Gunther, W. Preiser, S. van der Werf, H. R. Brodt, S. Becker, H. Rabenau, M. Panning, L. Kolesnikova, R. A. Fouchier, A. Berger, A. M. Burguiere, J. Cinatl, M. Eickmann, N. Escriou, K. Grywna, S. Kramme, J. C. Manuguerra, S. Muller, V. Rickerts, M. Sturmer, S. Vieth, H. D. Klenk, A. D. Osterhaus, H. Schmitz, and H. W. Doerr (2003). Identification of a novel coronavirus in patients with severe acute respiratory syndrome. N. Engl. J. Med. **348**:1967-1976.

Enserink M. (2003). Infectious diseases. SARS researchers report new animal models. Science **302**(5643):213.

González, J. M., Z. Pénzes, F. Almazán, E. Calvo, and L. Enjuanes (2002). Stabilization of a full-length infectious cDNA clone of transmissible gastroenteritis coronavirus by insertion of an intron. J. Virol. **76**:4655-4661.

Holmes, K. V., and L. Enjuanes (2003). Virology. The SARS coronavirus: a postgenomic era. Science **300**:1377-1378.

Izeta, A., Smerdou, C., Alonso, S., Penzes, Z., Méndez, A., Plana-Durán, J., and Enjuanes, L. (1999). Replication and packaging of transmissible gastroenteritis coronavirus-derived synthetic minigenomes. *J. Virol.* **73**, 1535-1545.

Kiyono et al. (1996). Mucosal Vaccines. Academic Press, New York.

Kuiken T, van den Hoogen BG, van Riel DA, Laman JD, van Amerongen G, Sprong L, Fouchier RA, Osterhaus AD. (2004). Experimental human metapneumovirus infection of cynomolgus macaques (Macaca fascicularis) results in virus replication in ciliated epithelial cells and pneumocytes with associated lesions throughout the respiratory tract. Am J Pathol. **164**:1893.

Marra, M. A., S. J. Jones, C. R. Astell, R. A. Holt, A. Brooks-Wilson, Y. S. Butterfield, J. Khattra, J. K. Asano, S. A. Barber, S. Y. Chan, A. Cloutier, S. M. Coughlin, D. Freeman, N. Girn, O. L. Griffith, S. R. Leach, M. Mayo, H. McDonald, S. B. Montgomery, P. K. Pandoh, A. S. Petrescu, A. G. Robertson, J. E. Schein, A. Siddiqui, D. E. Smailus, J. M. Stott, G. S. Yang, F. Plummer, A. Andonov, H. Artsob, N. Bastien, K. Bernard, T. F. Booth, D. Bowness, M. Czub, M. Drebot, L. Fernando, R. Flick, M. Garbutt, M. Gray, A. Grolla, S. Jones, H. Feldmann, A. Meyers, A. Kabani, Y. Li, S. Normand, U. Stroher, G. A. Tipples, S. Tyler, R. Vogrig, D. Ward, B. Watson, R. C. Brunham, M. Krajden, M. Petric, D. M. Skowronski, C. Upton, and R. L. Roper (2003). The genome sequence of the SARS-associated coronavirus. Science **300**:1399-1404.

Martina BE, Haagmans BL, Kuiken T, Fouchier RA, Rimmelzwaan GF, Van Amerongen G, Peiris JS, Lim W, Osterhaus AD. (2003). Virology: SARS virus infection of cats and ferrets. Nature **425**:915.

Rota, P. A., M. S. Oberste, S. S. Monroe, W. A. Nix, R. Campagnoli, J. P. Icenogle, S. Penaranda, B. Bankamp, K. Maher, M. H. Chen, S. Tong, A. Tamin, L. Lowe, M. Frace, J. L. De-Risi, Q. Chen, D. Wang, D. D. Erdman, T. C. Peret, C. Burns, T. G. Ksiazek, P. E. Rollin, A. Sanchez, S. Liffick, B. Holloway, J. Limor, K. McCaustland, M. Olsen-Rasmussen, R. Fouchier, S. Gunther, A. D. Osterhaus, C. Drosten, M. A. Pallansch, L. J. Anderson, and W. J. Bellini (2003). Characterization of a novel coronavirus associated with severe acute respiratory syndrome. Science **300**:1394-1399.

Sawicki, S. G., and D. L. Sawicki (1998). A new model for coronavirus transcription. Adv. Exp. Med. Biol. **440**:215-219.

Sawicki & Sawicki (1990). Coronavirus transcription: subgenomic mouse hepatitis virus replicative intermediates function in RNA synthesis. J Virol. **64**(3):1050-6.

Sethna, P. B., Hung, S.-L., and Brian, D. A. (1989). Coronavirus subgenomic minus-strand RNAs and the potential for mRNA replicons. *Proc. Natl. Acad. Sci. USA* **86**, 5626-5630.

Siddell, S. G. (1995). "The *Coronaviridae."* The Viruses (H. Fraenkel-Conrat, and R. R. Wagner, Eds.) Plenum Press, New York.

Sánchez, C. M., Izeta, A., Sánchez-Morgado, J. M., Alonso, S., Sola, I., Balasch, M., Plana-Durán, J. and Enjuanes, L. (1999). Targeted recombination demonstrates that the spike gene of transmissible gastroenteritis coronavirus is a determinant of its enteric tropism and virulence. J. Virol. **73**:7607-7618.

ter Meulen J, Bakker AB, van den Brink EN, Weverling GJ, Martina BE, Haagmans BL, Kuiken T, de Kruif J, Preiser W, Spaan W, Gelderblom HR, Goudsmit J, Osterhaus AD. (2004). Human monoclonal antibody as prophylaxis for SARS coronavirus infection in ferrets. Lancet. **26**;363(9427):2139-41.

Thiel, V., J. Herold, B. Schelle, and S. G. Siddell (2001). Infectious RNA transcribed *in vitro* from a cDNA copy of the human coronavirus genome cloned in vaccinia virus. J. Gen. Virol. **82**:1273-1281.

Thiel, V., K. A. Ivanov, A. Putics, T. Hertzig, B. Schelle, S. Bayer, B. Weibrich, E. J. Snijder, H. Rabenau, H. W. Doerr, A. E. Gorbalenya and J. Ziebuhr (2003). Mechanism and enzymes involved in SARS coronavirus genome expression. J. Gen. Virol. **84**: 2305-2315.

Toka, F.N. et al. (2004). Molecular adjuvants for mucosal immunity. Immunol Rev. **199**:100-112.

van der Most, R. G., and Spaan, W. J. M. (1995). Coronavirus replication, transcription, and RNA recombination. *In* "The Coronaviridae" (S. G. Siddell, Ed.), pp. 11-31. Plenum Press, New York.

Wang, K., C. Boysen, H. Shizuya, M. I. Simon and L. Hoods (1997). Complete nucleotide sequence of two generations of a bacterial artificial chromosome cloning vector. BioTechniques **23**: 992-994.

Yang ZY, Kong WP, Huang Y, Roberts A, Murphy BR, Subbarao K, Nabel GJ. (2004). A DNA vaccine induces SARS coronavirus neutralization and protective immunity in mice. Am J Pathol. **164**:1893-900.

Yount, B., K. M. Curtis, and R. S. Baric (2000). Strategy for systematic assembly of large RNA and DNA genomes: transmissible gastroenteritis virus model. J. Virol. **74**:10600-10611.

Yount, B., M. R. Denison, S. R. Weiss, and R. S. Baric (2002). Systematic assembly of a full-length infectious cDNA of mouse hepatitis virus strain A59. J. Virol. **76**:11065-11078.

Yount, B., K. M. Curtis, E. A. Fritz, L. E. Hensley, P. B. Jahrling, E. Prentice, M. R. Denison, T. W. Geisbert, and R. S. Baric (2003). Reverse genetics with a full-length infectious cDNA of severe acute respiratory syndrome coronavirus. Proc. Natl. Acad. Sci. USA **100**:12995-13000.

Ziebuhr, J., E. J. Snijder, and A. E. Gorbalenya (2000). Virus-encoded proteinases and proteolytic processing in the Nidovirales. J. Gen. Virol. **81**:853-879.

Zúñiga, S., I. Sola, S. Alonso, and L. Enjuanes (2004). Sequence motifs involved in the regulation of discontinuous coronavirus subgenomic RNA synthesis. J. Virol. **78**:980-994.

## Claims

1. Vaccine comprising a virus particle comprising a nucleic acid and one or several coat proteins, wherein
(a) the nucleic acid encodes SARS-CoV replicase, SARS-CoV N protein and at least one further SARS-CoV protein; and
(b) the one or several coat proteins are SARS-CoV coat proteins or proteins with a homology of at least 60% to SARS-CoV coat proteins necessary to allow the virus particle to infect human or animal cells,
wherein the nucleic acid is replication competent but not infectious.

2. Vaccine according to claim 1, wherein the SARS-CoV replicase is encoded by SEQ ID NO: 1 (Repla) and SEQ ID NO:2 (Rep1b) or a nucleic acid having a homology of at least 60% to the SEQ ID NOs: 1 or 2.

3. Vaccine according to claim 1 or 2, wherein the SARS-CoV N protein is encoded by SEQ ID NO: 3 or a nucleic acid having a homology of at least 60% to the SEQ ID NO: 3.

4. Vaccine according to claims 1 to 3, wherein the nucleic acid further encodes SARS-CoV spike protein.

5. Vaccine according to claim 4, wherein the SARS-CoV spike protein is encoded by SEQ ID NO: 4 or a nucleic acid having a homology of at least 60% to the SEQ ID NO: 4.

6. Vaccine according to claim 5, wherein the nucleic acid does not encode any other SARS-CoV protein except for SARS-CoV replicase, SARS-CoV N protein and SARS-CoV spike protein.

7. Vaccine according to claims 1 to 4, wherein the nucleic acid further encodes SARS-CoV M protein.

8. Vaccine according to claim 7, wherein the SARS-CoV M protein is encoded by SEQ ID NO: 5 or a nucleic acid having a homology of at least 60% to the SEQ ID NO: 5.

9. Vaccine according to claim 8, wherein the nucleic acid does not encode any other SARS-CoV protein except for SARS-CoV replicase, SARS-CoV N protein and SARS-CoV M protein.

10. Vaccine according to claims 1 to 4, wherein the nucleic acid further encodes SARS-CoV E protein.

11. Vaccine according to claim 10, wherein the SARS-CoV E protein is encoded by SEQ ID NO: 6 or a nucleic acid having a homology of at least 60% to the SEQ ID NO: 6.

12. Vaccine according to claim 11, wherein the nucleic acid does not encode any other SARS-CoV protein except for SARS-CoV replicase, SARS-CoV N protein and SARS-CoV E protein.

13. Vaccine according to claims 1 to 3 wherein the nucleic acid further encodes SARS-CoV M protein, SARS-CoV E protein and SARS-CoV spike protein.

14. Vaccine according to claim 13, wherein the nucleic acid does not encode any other SARS-CoV protein except for SARS-CoV replicase, SARS-CoV N protein, SARS-CoV M protein, SARS-CoV E protein and SARS-CoV spike protein.

15. Vaccine according to claims 1 to 14, wherein the nucleic acid encodes SARS-CoV proteins capable of associating into SARS-CoV virus like particles.

16. Vaccine according to claim 15, wherein the nucleic acid encodes SARS-CoV protein M, SARS-CoV spike protein and SARS-CoV protein E or proteins with a homology of at least 60% to SARS-CoV M and SARS-CoV E protein.

17. Vaccine according to claims 1 to 16, wherein administration of the vaccine to a mammal gives rise to an immune response that reduces or eliminates the disease symptoms caused by infection with the SARS virus.

18. Vaccine according to claims 1 to 17, wherein the vaccine is administered to a human or an animal.

19. Vaccine according to claim 18 wherein the vaccine is administered intramuscularly, intranvenously, subcutaneously or intranasally.

20. Vaccine according to claims 1 to 19, wherein the vaccine further comprises pharmaceutically acceptable carrier and/or adjuvants.

21. Vaccine according to claim 20, wherein the adjuvants is selected from the list consisting of cytokines, chemokines granulocyte/macrophage colony-stimulating factor, interleukin-2 (IL-2), IL-12, IL-18, IL-15 and IL-23.


SEQUENCE LISTING

<110> Consejo Superior de Investigaciones Cienticicas

<120> SARS Vaccine

<130> P 66798

<160> 10

<170> PatentIn version 3.1

<210> 1

<211> 13149

<212> DNA

<213> SARS-CoV

<400> 1
atggagagcc ttgttcttgg tgtcaacgag aaaacacacg tccaactcag tttgcctgtc 60

cttcaggtta gagacgtgct agtgcgtggc ttcggggact ctgtggaaga ggccctatcg 120

gaggcacgtg aacacctcaa aaatggcact tgtggtctag tagagctgga aaaaggcgta 180

ctgccccagc ttgaacagcc ctatgtgttc attaaacgtt ctgatgcctt aagcaccaat 240

cacggccaca aggtcgttga gctggttgca gaaatggacg gcattcagta cggtcgtagc 300

ggtataacac tgggagtact cgtgccacat gtgggcgaaa ccccaattgc ataccgcaat 360

gttcttcttc gtaagaacgg taataaggga gccggtggtc atagctatgg catcgatcta 420

aagtcttatg acttaggtga cgagcttggc actgatccca ttgaagatta tgaacaaaac 480

tggaacacta agcatggcag tggtgcactc cgtgaactca ctcgtgagct caatggaggt 540

gcagtcactc gctatgtcga caacaatttc tgtggcccag atgggtaccc tcttgattgc 600

atcaaagatt ttctcgcacg cgcgggcaag tcaatgtgca ctctttccga acaacttgat 660

tacatcgagt cgaagagagg tgtctactgc tgccgtgacc atgagcatga aattgcctgg 720

ttcactgagc gctctgataa gagctacgag caccagacac ccttcgaaat taagagtgcc 780

aagaaatttg acactttcaa aggggaatgc ccaaagtttg tgtttcctct taactcaaaa 840

gtcaaagtca ttcaaccacg tgttgaaaag aaaaagactg agggtttcat ggggcgtata 900

cgctctgtgt accctgttgc atctccacag gagtgtaaca atatgcactt gtctaccttg 960

atgaaatgta atcattgcga tgaagtttca tggcagacgt gcgactttct gaaagccact 1020

tgtgaacatt gtggcactga aaatttagtt attgaaggac ctactacatg tgggtaccta 1080

cctactaatg ctgtagtgaa aatgccatgt cctgcctgtc aagacccaga gattggacct 1140
gagcatagtg ttgcagatta tcacaaccac tcaaacattg aaactcgact ccgcaaggga 1200
ggtaggacta gatgttttgg aggctgtgtg tttgcctatg ttggctgcta taataagcgt 1260
gcctactggg ttcctcgtgc tagtgctgat attggctcag gccatactgg cattactggt 1320
gacaatgtgg agaccttgaa tgaggatctc cttgagatac tgagtcgtga acgtgttaac 1380
attaacattg ttggcgattt tcatttgaat gaagaggttg ccatcatttt ggcatctttc 1440
tctgcttcta caagtgcctt tattgacact ataaagagtc ttgattacaa gtctttcaaa 1500
accattgttg agtcctgcgg taactataaa gttaccaagg gaaagcccgt aaaaggtgct 1560
tggaacattg gacaacagag atcagtttta acaccactgt gtggttttcc ctcacaggct 1620
gctggtgtta tcagatcaat ttttgcgcgc acacttgatg cagcaaacca ctcaattcct 1680
gatttgcaaa gagcagctgt caccatactt gatggtattt ctgaacagtc attacgtctt 1740
gtcgacgcca tggtttatac ttcagacctg ctcaccaaca gtgtcattat tatggcatat 1800
gtaactggtg gtcttgtaca acagacttct cagtggttgt ctaatctttt gggcactact 1860
gttgaaaaac tcaggcctat ctttgaatgg attgaggcga aacttagtgc aggagttgaa 1920
tttctcaagg atgcttggga gattctcaaa tttctcatta caggtgtttt tgacatcgtc 1980
aagggtcaaa tacaggttgc ttcagataac atcaaggatt gtgtaaaatg cttcattgat 2040
gttgttaaca aggcactcga aatgtgcatt gatcaagtca ctatcgctgg cgcaaagttg 2100
cgatcactca acttaggtga agtcttcatc gctcaaagca agggacttta ccgtcagtgt 2160
atacgtggca aggagcagct gcaactactc atgcctctta aggcaccaaa agaagtaacc 2220
tttcttgaag gtgattcaca tgacacagta cttacctctg aggaggttgt tctcaagaac 2280
ggtgaactcg aagcactcga gacgcccgtt gatagcttca caaatggagc tatcgttggc 2340
acaccagtct gtgtaaatgg cctcatgctc ttagagatta aggacaaaga acaatactgc 2400
gcattgtctc ctggtttact ggctacaaac aatgtctttc gcttaaaagg gggtgcacca 2460
attaaaggtg taacctttgg agaagatact gtttgggaag ttcaaggtta caagaatgtg 2520
agaatcacat ttgagcttga tgaacgtgtt gacaaagtgc ttaatgaaaa gtgctctgtc 2580
tacactgttg aatccggtac cgaagttact gagtttgcat gtgttgtagc agaggctgtt 2640
gtgaagactt tacaaccagt ttctgatctc cttaccaaca tgggtattga tcttgatgag 2700
tggagtgtag ctacattcta cttatttgat gatgctggtg aagaaaactt ttcatcacgt 2760
atgtattgtt ccttttaccc tccagatgag gaagaagagg acgatgcaga gtgtgaggaa 2820
gaagaaattg atgaaacctg tgaacatgag tacggtacag aggatgatta tcaaggtctc 2880

cctctggaat ttggtgcctc agctgaaaca gttcgagttg aggaagaaga agaggaagac 2940

tggctggatg atactactga gcaatcagag attgagccag aaccagaacc tacacctgaa 3000

gaaccagtta atcagtttac tggttattta aaacttactg acaatgttgc cattaaatgt 3060

gttgacatcg ttaaggaggc acaaagtgct aatcctatgg tgattgtaaa tgctgctaac 3120

atacacctga aacatggtgg tggtgtagca ggtgcactca acaaggcaac caatggtgcc 3180

atgcaaaagg agagtgatga ttacattaag ctaaatggcc ctcttacagt aggagggtct 3240

tgtttgcttt ctggacataa tcttgctaag aagtgtctgc atgttgttgg acctaaccta 3300

aatgcaggtg aggacatcca gcttcttaag gcagcatatg aaaatttcaa ttcacaggac 3360

atcttacttg caccattgtt gtcagcaggc atatttggtg ctaaaccact tcagtcttta 3420

caagtgtgcg tgcagacggt tcgtacacag gtttatattg cagtcaatga caaagctctt 3480

tatgagcagg ttgtcatgga ttatcttgat aacctgaagc ctagagtgga agcacctaaa 3540

caagaggagc caccaaacac agaagattcc aaaactgagg agaaatctgt cgtacagaag 3600

cctgtcgatg tgaagccaaa aattaaggcc tgcattgatg aggttaccac aacactggaa 3660

gaaactaagt ttcttaccaa taagttactc ttgtttgctg atatcaatgg taagctttac 3720

catgattctc agaacatgct tagaggtgaa gatatgtctt tccttgagaa ggatgcacct 3780

tacatggtag gtgatgttat cactagtggt gatatcactt gtgttgtaat accctccaaa 3840

aaggctggtg gcactactga gatgctctca agagctttga agaaagtgcc agttgatgag 3900

tatataacca cgtaccctgg acaaggatgt gctggttata cacttgagga agctaagact 3960

gctcttaaga aatgcaaatc tgcattttat gtactacctt cagaagcacc taatgctaag 4020

gaagagattc taggaactgt atcctggaat ttgagagaaa tgcttgctca tgctgaagag 4080

acaagaaaat taatgcctat atgcatggat gttagagcca taatggcaac catccaacgt 4140

aagtataaag gaattaaaat tcaagagggc atcgttgact atggtgtccg attcttcttt 4200

tatactagta aagagcctgt agcttctatt attacgaagc tgaactctct aaatgagccg 4260

cttgtcacaa tgccaattgg ttatgtgaca catggtttta atcttgaaga ggctgcgcgc 4320

tgtatgcgtt ctcttaaagc tcctgccgta gtgtcagtat catcaccaga tgctgttact 4380

acatataatg gatacctcac ttcgtcatca aagacatctg aggagcactt tgtagaaaca 4440

gtttctttgg ctggctctta cagagattgg tcctattcag gacagcgtac agagttaggt 4500

gttgaatttc ttaagcgtgg tgacaaaatt gtgtaccaca ctctggagag ccccgtcgag 4560

tttcatcttg acggtgaggt tctttcactt gacaaactaa agagtctctt atccctgcgg 4620

gaggttaaga ctataaaagt gttcacaact gtggacaaca ctaatctcca cacacagctt 4680

gtggatatgt ctatgacata tggacagcag tttggtccaa catacttgga tggtgctgat 4740
gttacaaaaa ttaaacctca tgtaaatcat gagggtaaga ctttctttgt actacctagt 4800
gatgacacac tacgtagtga agctttcgag tactaccata ctcttgatga gagttttctt 4860
ggtaggtaca tgtctgcttt aaaccacaca aagaaatgga aatttcctca agttggtggt 4920
ttaacttcaa ttaaatgggc tgataacaat tgttatttgt ctagtgtttt attagcactt 4980
caacagcttg aagtcaaatt caatgcacca gcacttcaag aggcttatta tagagcccgt 5040
gctggtgatg ctgctaactt ttgtgcactc atactcgctt acagtaataa aactgttggc 5100
gagcttggtg atgtcagaga aactatgacc catcttctac agcatgctaa tttggaatct 5160
gcaaagcgag ttcttaatgt ggtgtgtaaa cattgtggtc agaaaactac taccttaacg 5220
ggtgtagaag ctgtgatgta tatgggtact ctatcttatg ataatcttaa gacaggtgtt 5280
tccattccat gtgtgtgtgg tcgtgatgct acacaatatc tagtacaaca agagtcttct 5340
tttgttatga tgtctgcacc acctgctgag tataaattac agcaaggtac attcttatgt 5400
gcgaatgagt acactggtaa ctatcagtgt ggtcattaca ctcatataac tgctaaggag 5460
accctctatc gtattgacgg agctcacctt acaaagatgt cagagtacaa aggaccagtg 5520
actgatgttt tctacaagga aacatcttac actacaacca tcaagcctgt gtcgtataaa 5580
ctcgatggag ttacttacac agagattgaa ccaaaattgg atgggtatta taaaaaggat 5640
aatgcttact atacagagca gcctatagac cttgtaccaa ctcaaccatt accaaatgcg 5700
agttttgata atttcaaact cacatgttct aacacaaaat ttgctgatga tttaaatcaa 5760
atgacaggct tcacaaagcc agcttcacga gagctatctg tcacattctt cccagacttg 5820
aatggcgatg tagtggctat tgactataga cactattcag cgagtttcaa gaaaggtgct 5880
aaattactgc ataagccaat tgtttggcac attaaccagg ctacaaccaa gacaacgttc 5940
aaaccaaaca cttggtgttt acgttgtctt tggagtacaa agccagtaga tacttcaaat 6000
tcatttgaag ttctggcagt agaagacaca caaggaatgg acaatcttgc ttgtgaaagt 6060
caacaaccca cctctgaaga agtagtggaa aatcctacca tacagaagga agtcatagag 6120
tgtgacgtga aaactaccga agttgtaggc aatgtcatac ttaaaccatc agatgaaggt 6180
gttaaagtaa cacaagagtt aggtcatgag gatcttatgg ctgcttatgt ggaaaacaca 6240
agcattacca ttaagaaacc taatgagctt tcactagcct taggtttaaa aacaattgcc 6300
actcatggta ttgctgcaat taatagtgtt ccttggagta aaattttggc ttatgtcaaa 6360
ccattcttag gacaagcagc aattacaaca tcaaattgcg ctaagagatt agcacaacgt 6420
gtgtttaaca attatatgcc ttatgtgttt acattattgt tccaattgtg tacttttact 6480

aaaagtacca attctagaat tagagcttca ctacctacaa ctattgctaa aaatagtgtt 6540
aagagtgttg ctaaattatg tttggatgcc ggcattaatt atgtgaagtc acccaaattt 6600
tctaaattgt tcacaatcgc tatgtggcta ttgttgttaa gtatttgctt aggttctcta 6660
atctgtgtaa ctgctgcttt tggtgtactc ttatctaatt ttggtgctcc ttcttattgt 6720
aatggcgtta gagaattgta tcttaattcg tctaacgtta ctactatgga tttctgtgaa 6780
ggttcttttc cttgcagcat ttgtttaagt ggattagact cccttgattc ttatccagct 6840
cttgaaacca ttcaggtgac gatttcatcg tacaagctag acttgacaat tttaggtctg 6900
gccgctgagt gggttttggc atatatgttg ttcacaaaat tcttttattt attaggtctt 6960
tcagctataa tgcaggtgtt ctttggctat tttgctagtc atttcatcag caattcttgg 7020
ctcatgtggt ttatcattag tattgtacaa atggcacccg tttctgcaat ggttaggatg 7080
tacatcttct ttgcttcttt ctactacata tggaagagct atgttcatat catggatggt 7140
tgcacctctt cgacttgcat gatgtgctat aagcgcaatc gtgccacacg cgttgagtgt 7200
acaactattg ttaatggcat gaagagatct ttctatgtct atgcaaatgg aggccgtggc 7260
ttctgcaaga ctcacaattg gaattgtctc aattgtgaca cattttgcac tggtagtaca 7320
ttcattagtg atgaagttgc tcgtgatttg tcactccagt ttaaaagacc aatcaaccct 7380
actgaccagt catcgtatat tgttgatagt gttgctgtga aaaatggcgc gcttcacctc 7440
tactttgaca aggctggtca aaagacctat gagagacatc cgctctccca ttttgtcaat 7500
ttagacaatt tgagagctaa caacactaaa ggttcactgc ctattaatgt catagttttt 7560
gatggcaagt ccaaatgcga cgagtctgct tctaagtctg cttctgtgta ctacagtcag 7620
ctgatgtgcc aacctattct gttgcttgac caagttcttg tatcagacgt tggagatagt 7680
actgaagttt ccgttaagat gtttgatgct tatgtcgaca ccttttcagc aacttttagt 7740
gttcctatgg aaaaacttaa ggcacttgtt gctacagctc acagcgagtt agcaaagggt 7800
gtagctttag atggtgtcct ttctacattc gtgtcagctg cccgacaagg tgttgttgat 7860
accgatgttg acacaaagga tgttattgaa tgtctcaaac tttcacatca ctctgactta 7920
gaagtgacag gtgacagttg taacaatttc atgctcacct ataataaggt tgaaaacatg 7980
acgcccagag atcttggcgc atgtattgac tgtaatgcaa ggcatatcaa tgcccaagta 8040
gcaaaaagtc acaatgtttc actcatctgg aatgtaaaag actacatgtc tttatctgaa 8100
cagctgcgta aacaaattcg tagtgctgcc aagaagaaca acataccttt tagactaact 8160
tgtgctacaa ctagacaggt tgtcaatgtc ataactacta aaatctcact caagggtggt 8220
aagattgtta gtacttgttt taaacttatg cttaaggcca cattattgtg cgttcttgct 8280

gcattggttt gttatatcgt tatgccagta catacattgt caatccatga tggttacaca 8340
aatgaaatca ttggttacaa agccattcag gatggtgtca ctcgtgacat catttctact 8400
gatgattgtt ttgcaaataa acatgctggt tttgacgcat ggtttagcca gcgtggtggt 8460
tcatacaaaa atgacaaaag ctgccctgta gtagctgcta tcattacaag agagattggt 8520
ttcatagtgc ctggcttacc gggtactgtg ctgagagcaa tcaatggtga cttcttgcat 8580
tttctacctc gtgtttttag tgctgttggc aacatttgct acacaccttc caaactcatt 8640
gagtatagtg attttgctac ctctgcttgc gttcttgctg ctgagtgtac aatttttaag 8700
gatgctatgg gcaaacctgt gccatattgt tatgacacta atttgctaga gggttctatt 8760
tcttatagtg agcttcgtcc agacactcgt tatgtgctta tggatggttc catcatacag 8820
tttcctaaca cttacctgga gggttctgtt agagtagtaa caacttttga tgctgagtac 8880
tgtagacatg gtacatgcga aaggtcagaa gtaggtattt gcctatctac cagtggtaga 8940
tgggttctta ataatgagca ttacagagct ctatcaggag ttttctgtgg tgttgatgcg 9000
atgaatctca tagctaacat ctttactcct cttgtgcaac ctgtgggtgc tttagatgtg 9060
tctgcttcag tagtggctgg tggtattatt gccatattgg tgacttgtgc tgcctactac 9120
tttatgaaat tcagacgtgt ttttggtgag tacaaccatg ttgttgctgc taatgcactt 9180
ttgtttttga tgtctttcac tatactctgt ctggtaccag cttacagctt tctgccggga 9240
gtctactcag tcttttactt gtacttgaca ttctatttca ccaatgatgt ttcattcttg 9300
gctcaccttc aatggtttgc catgttttct cctattgtgc ctttttggat aacagcaatc 9360
tatgtattct gtatttctct gaagcactgc cattggttct ttaacaacta tcttaggaaa 9420
agagtcatgt ttaatggagt tacatttagt accttcgagg aggctgcttt gtgtaccttt 9480
ttgctcaaca aggaaatgta cctaaaattg cgtagcgaga cactgttgcc acttacacag 9540
tataacaggt atcttgctct atataacaag tacaagtatt tcagtggagc cttagatact 9600
accagctatc gtgaagcagc ttgctgccac ttagcaaagg ctctaaatga ctttagcaac 9660
tcaggtgctg atgttctcta ccaaccacca cagacatcaa tcacttctgc tgttctgcag 9720
agtggtttta ggaaaatggc attcccgtca ggcaaagttg aagggtgcat ggtacaagta 9780
acctgtggaa ctacaactct taatggattg tggttggatg acacagtata ctgtccaaga 9840
catgtcattt gcacagcaga agacatgctt aatcctaact atgaagatct gctcattcgc 9900
aaatccaacc atagctttct tgttcaggct ggcaatgttc aacttcgtgt tattggccat 9960
tctatgcaaa attgtctgct taggcttaaa gttgatactt ctaaccctaa gacacccaag 10020
tataaatttg tccgtatcca acctggtcaa acattttcag ttctagcatg ctacaatggt 10080

tcaccatctg gtgtttatca gtgtgccatg agacctaatc ataccattaa aggttctttc 10140
cttaatggat catgtggtag tgttggtttt aacattgatt atgattgcgt gtctttctgc 10200
tatatgcatc atatggagct tccaacagga gtacacgctg gtactgactt agaaggtaaa 10260
ttctatggtc catttgttga cagacaaact gcacaggctg caggtacaga cacaaccata 10320
acattaaatg ttttggcatg gctgtatgct gctgttatca atggtgatag gtggtttctt 10380
aatagattca ccactacttt gaatgacttt aaccttgtgg caatgaagta caactatgaa 10440
cctttgacac aagatcatgt tgacatattg ggacctcttt ctgctcaaac aggaattgcc 10500
gtcttagata tgtgtgctgc tttgaaagag ctgctgcaga atggtatgaa tggtcgtact 10560
atccttggta gcactatttt agaagatgag tttacaccat ttgatgttgt tagacaatgc 10620
tctggtgtta ccttccaagg taagttcaag aaaattgtta agggcactca tcattggatg 10680
cttttaactt tcttgacatc actattgatt cttgttcaaa gtacacagtg gtcactgttt 10740
ttctttgttt acgagaatgc tttcttgcca tttactcttg gtattatggc aattgctgca 10800
tgtgctatgc tgcttgttaa gcataagcac gcattcttgt gcttgtttct gttaccttct 10860
cttgcaacag ttgcttactt taatatggtc tacatgcctg ctagctgggt gatgcgtatc 10920
atgacatggc ttgaattggc tgacactagc ttgtctggtt ataggcttaa ggattgtgtt 10980
atgtatgctt cagctttagt tttgcttatt ctcatgacag ctcgcactgt ttatgatgat 11040
gctgctagac gtgtttggac actgatgaat gtcattacac ttgtttacaa agtctactat 11100
ggtaatgctt tagatcaagc tatttccatg tgggccttag ttatttctgt aacctctaac 11160
tattctggtg tcgttacgac tatcatgttt ttagctagag ctatagtgtt tgtgtgtgtt 11220
gagtattacc cattgttatt tattactggc aacaccttac agtgtatcat gcttgtttat 11280
tgtttcttag gctattgttg ctgctgctac tttggccttt tctgtttact caaccgttac 11340
ttcaggctta ctcttggtgt ttatgactac ttggtctcta cacaagaatt taggtatatg 11400
aactcccagg ggcttttgcc tcctaagagt agtattgatg ctttcaagct taacattaag 11460
ttgttgggta ttggaggtaa accatgtatc aaggttgcta ctgtacagtc taaaatgtct 11520
gacgtaaagt gcacatctgt ggtactgctc tcggttcttc aacaacttag agtagagtca 11580
tcttctaaat tgtgggcaca atgtgtacaa ctccacaatg atattcttct tgcaaaagac 11640
acaactgaag ctttcgagaa gatggtttct cttttgtctg ttttgctatc catgcagggt 11700
gctgtagaca ttaataggtt gtgcgaggaa atgctcgata accgtgctac tcttcaggct 11760
attgcttcag aatttagttc tttaccatca tatgccgctt atgccactgc ccaggaggcc 11820
tatgagcagg ctgtagctaa tggtgattct gaagtcgttc tcaaaaagtt aaagaaatct 11880

ttgaatgtgg ctaaatctga gtttgaccgt gatgctgcca tgcaacgcaa gttggaaaag 11940

atggcagatc aggctatgac ccaaatgtac aaacaggcaa gatctgagga caagagggca 12000

aaagtaacta gtgctatgca aacaatgctc ttcactatgc ttaggaagct tgataatgat 12060

gcacttaaca acattatcaa caatgcgcgt gatggttgtg ttccactcaa catcatacca 12120

ttgactacag cagccaaact catggttgtt gtccctgatt atggtaccta caagaacact 12180

tgtgatggta acacctttac atatgcatct gcactctggg aaatccagca agttgttgat 12240

gcggatagca agattgttca acttagtgaa attaacatgg acaattcacc aaatttggct 12300

tggcctctta ttgttacagc tctaagagcc aactcagctg ttaaactaca gaataatgaa 12360

ctgagtccag tagcactacg acagatgtcc tgtgcggctg gtaccacaca aacagcttgt 12420

actgatgaca atgcacttgc ctactataac aattcgaagg gaggtaggtt tgtgctggca 12480

ttactatcag accaccaaga tctcaaatgg gctagattcc ctaagagtga tggtacaggt 12540

acaatttaca cagaactgga accaccttgt aggtttgtta cagacacacc aaaagggcct 12600

aaagtgaaat acttgtactt catcaaaggc ttaaacaacc taaatagagg tatggtgctg 12660

ggcagtttag ctgctacagt acgtcttcag gctggaaatg ctacagaagt acctgccaat 12720

tcaactgtgc tttccttctg tgcttttgca gtagaccctg ctaaagcata taaggattac 12780

ctagcaagtg gaggacaacc aatcaccaac tgtgtgaaga tgttgtgtac acacactggt 12840

acaggacagg caattactgt aacaccagaa gctaacatgg accaagagtc ctttggtggt 12900

gcttcatgtt gtctgtattg tagatgccac attgaccatc caaatcctaa aggattctgt 12960

gacttgaaag gtaagtacgt ccaaatacct accacttgtg ctaatgaccc agtgggtttt 13020

acacttagaa acacagtctg taccgtctgc ggaatgtgga aaggttatgg ctgtagttgt 13080

gaccaactcc gcgaaccctt gatgcagtct gcggatgcat caacgttttt aaacgggttt 13140

gcggtgtaa 13149

<210> 2

<211> 8088

<212> DNA

<213> SARS-CoV

<400> 2
cgggtttgcg gtgtaagtgc agcccgtctt acaccgtgcg gcacaggcac tagtactgat 60

gtcgtctaca gggcttttga tatttacaac gaaaaagttg ctggttttgc aaagttccta 120

aaaactaatt gctgtcgctt ccaggagaag gatgaggaag gcaatttatt agactcttac 180

tttgtagtta agaggcatac tatgtctaac taccaacatg aagagactat ttataacttg 240

gttaaagatt gtccagcggt tgctgtccat gactttttca agtttagagt agatggtgac 300

atggtaccac atatatcacg tcagcgtcta actaaataca caatggctga tttagtctat 360

gctctacgtc attttgatga gggtaattgt gatacattaa aagaaatact cgtcacatac 420

aattgctgtg atgatgatta tttcaataag aaggattggt atgacttcgt agagaatcct 480

gacatcttac gcgtatatgc taacttaggt gagcgtgtac gccaatcatt attaaagact 540

gtacaattct gcgatgctat gcgtgatgca ggcattgtag gcgtactgac attagataat 600

caggatctta atgggaactg gtacgatttc ggtgatttcg tacaagtagc accaggctgc 660

ggagttccta ttgtggattc atattactca ttgctgatgc ccatcctcac tttgactagg 720

gcattggctg ctgagtccca tatggatgct gatctcgcaa aaccacttat taagtgggat 780

ttgctgaaat atgattttac ggaagagaga ctttgtctct tcgaccgtta ttttaaatat 840

tgggaccaga cataccatcc caattgtatt aactgtttgg atgataggtg tatccttcat 900

tgtgcaaact ttaatgtgtt attttctact gtgtttccac ctacaagttt tggaccacta 960

gtaagaaaaa tatttgtaga tggtgttcct tttgttgttt caactggata ccattttcgt 1020

gagttaggag tcgtacataa tcaggatgta aacttacata gctcgcgtct cagtttcaag 1080

gaacttttag tgtatgctgc tgatccagct atgcatgcag cttctggcaa tttattgcta 1140

gataaacgca ctacatgctt ttcagtagct gcactaacaa acaatgttgc ttttcaaact 1200

gtcaaacccg gtaattttaa taaagacttt tatgactttg ctgtgtctaa aggtttcttt 1260

aaggaaggaa gttctgttga actaaaacac ttcttctttg ctcaggatgg caacgctgct 1320

atcagtgatt atgactatta tcgttataat ctgccaacaa tgtgtgatat cagacaactc 1380

ctattcgtag ttgaagttgt tgataaatac tttgattgtt acgatggtgg ctgtattaat 1440

gccaaccaag taatcgttaa caatctggat aaatcagctg gtttcccatt taataaatgg 1500

ggtaaggcta gactttatta tgactcaatg agttatgagg atcaagatgc acttttcgcg 1560

tatactaagc gtaatgtcat ccctactata actcaaatga atcttaagta tgccattagt 1620

gcaaagaata gagctcgcac cgtagctggt gtctctatct gtagtactat gacaaataga 1680

cagtttcatc agaaattatt gaagtcaata gccgccacta gaggagctac tgtggtaatt 1740

ggaacaagca agttttacgg tggctggcat aatatgttaa aaactgttta cagtgatgta 1800

gaaactccac accttatggg ttgggattat ccaaaatgtg acagagccat gcctaacatg 1860

cttaggataa tggcctctct tgttcttgct cgcaaacata acacttgctg taacttatca 1920

caccgtttct acaggttagc taacgagtgt gcgcaagtat taagtgagat ggtcatgtgt 1980

ggcggctcac tatatgttaa accaggtgga acatcatccg gtgatgctac aactgcttat 2040
gctaatagtg tctttaacat ttgtcaagct gttacagcca atgtaaatgc acttctttca 2100
actgatggta ataagatagc tgacaagtat gtccgcaatc tacaacacag gctctatgag 2160
tgtctctata gaaataggga tgttgatcat gaattcgtgg atgagtttta cgcttacctg 2220
cgtaaacatt tctccatgat gattctttct gatgatgccg ttgtgtgcta taacagtaac 2280
tatgcggctc aaggtttagt agctagcatt aagaacttta aggcagttct ttattatcaa 2340
aataatgtgt tcatgtctga ggcaaaatgt tggactgaga ctgaccttac taaaggacct 2400
cacgaatttt gctcacagca tacaatgcta gttaaacaag gagatgatta cgtgtacctg 2460
ccttacccag atccatcaag aatattaggc gcaggctgtt ttgtcgatga tattgtcaaa 2520
acagatggta cacttatgat tgaaaggttc gtgtcactgg ctattgatgc ttacccactt 2580
acaaaacatc ctaatcagga gtatgctgat gtctttcact tgtatttaca atacattaga 2640
aagttacatg atgagcttac tggccacatg ttggacatgt attccgtaat gctaactaat 2700
gataacacct cacggtactg ggaacctgag ttttatgagg ctatgtacac accacataca 2760
gtcttgcagg ctgtaggtgc ttgtgtattg tgcaattcac agacttcact tcgttgcggt 2820
gcctgtatta ggagaccatt cctatgttgc aagtgctgct atgaccatgt catttcaaca 2880
tcacacaaat tagtgttgtc tgttaatccc tatgtttgca atgccccagg ttgtgatgtc 2940
actgatgtga cacaactgta tctaggaggt atgagctatt attgcaagtc acataagcct 3000
cccattagtt ttccattatg tgctaatggt caggtttttg gtttatacaa aaacacatgt 3060
gtaggcagtg acaatgtcac tgacttcaat gcgatagcaa catgtgattg gactaatgct 3120
ggcgattaca tacttgccaa cacttgtact gagagactca agcttttcgc agcagaaacg 3180
ctcaaagcca ctgaggaaac atttaagctg tcatatggta ttgctactgt acgcgaagta 3240
ctctctgaca gagaattgca tctttcatgg gaggttggaa aacctagacc accattgaac 3300
agaaactatg tctttactgg ttaccgtgta actaaaaata gtaaagtaca gattggagag 3360
tacacctttg aaaaaggtga ctatggtgat gctgttgtgt acagaggtac tacgacatac 3420
aagttgaatg ttggtgatta ctttgtgttg acatctcaca ctgtaatgcc acttagtgca 3480
cctactctag tgccacaaga gcactatgtg agaattactg gcttgtaccc aacactcaac 3540
atctcagatg agttttctag caatgttgca aattatcaaa aggtcggcat gcaaaagtac 3600
tctacactcc aaggaccacc tggtactggt aagagtcatt ttgccatcgg acttgctctc 3660
tattacccat ctgctcgcat agtgtatacg gcatgctctc atgcagctgt tgatgcccta 3720
tgtgaaaagg cattaaaata tttgcccata gataaatgta gtagaatcat acctgcgcgt 3780

gcgcgcgtag agtgttttga taaattcaaa gtgaattcaa cactagaaca gtatgttttc 3840
tgcactgtaa atgcattgcc agaaacaact gctgacattg tagtctttga tgaaatctct 3900
atggctacta attatgactt gagtgttgtc aatgctagac ttcgtgcaaa acactacgtc 3960
tatattggcg atcctgctca attaccagcc ccccgcacat tgctgactaa aggcacacta 4020
gaaccagaat attttaattc agtgtgcaga cttatgaaaa caataggtcc agacatgttc 4080
cttggaactt gtcgccgttg tcctgctgaa attgttgaca ctgtgagtgc tttagtttat 4140
gacaataagc taaaagcaca caaggataag tcagctcaat gcttcaaaat gttctacaaa 4200
ggtgttatta cacatgatgt ttcatctgca atcaacagac ctcaataggg cgttgtaaga 4260
gaatttctta cacgcaatcc tgcttggaga aaagctgttt ttatctcacc ttataattca 4320
cagaacgctg tagcttcaaa aatcttagga ttgcctacgc agactgttga ttcatcacag 4380
ggttctgaat atgactatgt catattcaca caaactactg aaacagcaca ctcttgtaat 4440
gtcaaccgct tcaatgtggc tatcacaagg gcaaaaattg gcattttgtg cataatgtct 4500
gatagagatc tttatgacaa actgcaattt acaagtctag aaataccacg tcgcaatgtg 4560
gctacattac aagcagaaaa tgtaactgga ctttttaagg actgtagtaa gatcattact 4620
ggtcttcatc ctacacaggc acctacacac ctcagcgttg atataaagtt caagactgaa 4680
ggattatgtg ttgacatacc aggcatacca aaggacatga cctaccgtag actcatctct 4740
atgatgggtt tcaaaatgaa ttaccaagtc aatggttacc ctaatatgtt tatcacccgc 4800
gaagaagcta ttcgtcacgt tcgtgcgtgg attggctttg atgtagaggg ctgtcatgca 4860
actagagatg ctgtgggtac taacctacct ctccagctag gattttctac aggtgttaac 4920
ttagtagctg taccgactgg ttatgttgac actgaaaata acacagaatt caccagagtt 4980
aatgcaaaac ctccaccagg tgaccagttt aaacatctta taccactcat gtataaaggc 5040
ttgccctgga atgtagtgcg tattaagata gtacaaatgc tcagtgatac actgaaagga 5100
ttgtcagaca gagtcgtgtt cgtcctttgg gcgcatggct ttgagcttac atcaatgaag 5160
tactttgtca agattggacc tgaaagaacg tgttgtctgt gtgacaaacg tgcaacttgc 5220
ttttctactt catcagatac ttatgcctgc tggaatcatt ctgtgggttt tgactatgtc 5280
tataacccat ttatgattga tgttcagcag tggggcttta cgggtaacct tcagagtaac 5340
catgaccaac attgccaggt acatggaaat gcacatgtgg ctagttgtga tgctatcatg 5400
actagatgtt tagcagtcca tgagtgcttt gttaagcgcg ttgattggtc tgttgaatac 5460
cctattatag gagatgaact gagggttaat tctgcttgca gaaaagtaca acacatggtt 5520
gtgaagtctg cattgcttgc tgataagttt ccagttcttc atgacattgg aaatccaaag 5580

gctatcaagt gtgtgcctca ggctgaagta gaatggaagt tctacgatgc tcagccatgt 5640
agtgacaaag cttacaaaat agaggagctc ttctattctt atgctacaca tcacgataaa 5700
ttcactgatg gtgtttgttt gttttggaat tgtaacgttg atcgttaccc agccaatgca 5760
attgtgtgta ggtttgacac aagagtcttg tcaaacttga acttaccagg ctgtgatggt 5820
ggtagtttgt atgtgaataa gcatgcattc cacactccag ctttcgataa aagtgcattt 5880
actaatttaa agcaattgcc tttcttttac tattctgata gtccttgtga gtctcatggc 5940
aaacaagtag tgtcggatat tgattatgtt ccactcaaat ctgctacgtg tattacacga 6000
tgcaatttag gtggtgctgt ttgcagacac catgcaaatg agtaccgaca gtacttggat 6060
gcatataata tgatgatttc tgctggattt agcctatgga tttacaaaca atttgatact 6120
tataacctgt ggaatacatt taccaggtta cagagtttag aaaatgtggc ttataatgtt 6180
gttaataaag gacactttga tggacacgcc ggcgaagcac ctgtttccat cattaataat 6240
gctgtttaca caaaggtaga tggtattgat gtggagatct ttgaaaataa gacaacactt 6300
cctgttaatg ttgcatttga gctttgggct aagcgtaaca ttaaaccagt gccagagatt 6360
aagatactca ataatttggg tgttgatatc gctgctaata ctgtaatctg ggactacaaa 6420
agagaagccc cagcacatgt atctacaata ggtgtctgca caatgactga cattgccaag 6480
aaacctactg agagtgcttg ttcttcactt actgtcttgt ttgatggtag agtggaagga 6540
caggtagacc tttttagaaa cgcccgtaat ggtgttttaa taacagaagg ttcagtcaaa 6600
ggtctaacac cttcaaaggg accagcacaa gctagcgtca atggagtcac attaattgga 6660
gaatcagtaa aaacacagtt taactacttt aagaaagtag acggcattat tcaacagttg 6720
cctgaaacct actttactca gagcagagac ttagaggatt ttaagcccag atcacaaatg 6780
gaaactgact ttctcgagct cgctatggat gaattcatac agcgatataa gctcgagggc 6840
tatgccttcg aacacatcgt ttatggagat ttcagtcatg gacaacttgg cggtcttcat 6900
ttaatgatag gcttagccaa gcgctcacaa gattcaccac ttaaattaga ggattttatc 6960
cctatggaca gcacagtgaa aaattacttc ataacagatg cgcaaacagg ttcatcaaaa 7020
tgtgtgtgtt ctgtgattga tcttttactt gatgactttg tcgagataat aaagtcacaa 7080
gatttgtcag tgatttcaaa agtggtcaag gttacaattg actatgctga aatttcattc 7140
atgctttggt gtaaggatgg acatgttgaa accttctacc caaaactaca agcaagtcaa 7200
gcgtggcaac caggtgttgc gatgcctaac ttgtacaaga tgcaaagaat gcttcttgaa 7260
aagtgtgacc ttcagaatta tggtgaaaat gctgttatac caaaaggaat aatgatgaat 7320
gtcgcaaagt atactcaact gtgtcaatac ttaaatacac ttactttagc tgtaccctac 7380

aacatgagag ttattcactt tggtgctggc tctgataaag gagttgcacc aggtacagct 7440

gtgctcagac aatggttgcc aactggcaca ctacttgtcg attcagatct taatgacttc 7500

gtctccgacg cagattctac tttaattgga gactgtgcaa cagtacatac ggctaataaa 7560

tgggacctta ttattagcga tatgtatgac cctaggacca aacatgtgac aaaagagaat 7620

gactctaaag aagggttttt cacttatctg tgtggattta taaagcaaaa actagccctg 7680

ggtggttcta tagctgtaaa gataacagag cattcttgga atgctgacct ttacaagctt 7740

atgggccatt tctcatggtg gacagctttt gttacaaatg taaatgcatc atcatcggaa 7800

gcatttttaa ttggggctaa ctatcttggc aagccgaagg aacaaattga tggctatacc 7860

atgcatgcta actacatttt ctggaggaac acaaatccta tccagttgtc ttcctattca 7920

ctctttgaca tgagcaaatt tcctcttaaa ttaagaggaa ctgctgtaat gtctcttaag 7980

gagaatcaaa tcaatgatat gatttattct cttctggaaa aaggtaggct tatcattaga 8040

gaaaacaaca gagttgtggt ttcaagtgat attcttgtta acaactaa 8088

<210> 3

<211> 1269

<212> DNA

<213> SARS-CoV

<400> 3
atgtctgata atggacccca atcaaaccaa cgtagtgccc cccgcattac atttggtgga 60

cccacagatt caactgacaa taaccagaat ggaggacgca atggggcaag gccaaaacag 120

cgccgacccc aaggtttacc caataatact gcgtcttggt tcacagctct cactcagcat 180

ggcaaggagg aacttagatt ccctcgaggc cagggcgttc caatcaacac caatagtggt 240

ccagatgacc aaattggcta ctaccgaaga gctacccgac gagttcgtgg tggtgacggc 300

aaaatgaaag agctcagccc cagatggtac ttctattacc taggaactgg cccagaagct 360

tcacttccct acggcgctaa caaagaaggc atcgtatggg ttgcaactga gggagccttg 420

aatacaccca aagaccacat tggcacccgc aatcctaata acaatgctgc caccgtgcta 480

caacttcctc aaggaacaac attgccaaaa ggcttctacg cagagggaag cagaggcggc 540

agtcaagcct cttctcgctc ctcatcacgt agtcgcggta attcaagaaa ttcaactcct 600

ggcagcagta ggggaaattc tcctgctcga atggctagcg gaggtggtga aactgccctc 660

gcgctattgc tgctagacag attgaaccag cttgagagca aagtttctgg taaaggccaa 720

caacaacaag gccaaactgt cactaagaaa tctgctgctg aggcatctaa aaagcctcgc 780

caaaaacgta ctgccacaaa acagtacaac gtcactcaag catttgggag acgtggtcca 840

gaacaaaccc aaggaaattt cggggaccaa gacctaatca gacaaggaac tgattacaaa 900

cattggccgc aaattgcaca atttgctcca agtgcctctg cattctttgg aatgtcacgc 960

attggcatgg aagtcacacc ttcgggaaca tggctgactt atcatggagc cattaaattg 1020

gatgacaaag atccacaatt caaagacaac gtcatactgc tgaacaagca cattgacgca 1080

tacaaaacat tcccaccaac agagcctaaa aaggacaaaa agaaaaagac tgatgaagct 1140

cagcctttgc cgcagagaca aaagaagcag cccactgtga ctcttcttcc tgcggctgac 1200

atggatgatt tctccagaca acttcaaaat tccatgagtg gagcttctgc tgattcaact 1260

caggcataa 1269

<210> 4

<211> 3768

<212> DNA

<213> SARS-CoV

<400> 4
atgtttattt tcttattatt tcttactctc actagtggta gtgaccttga ccggtgcacc 60

acttttgatg atgttcaagc tcctaattac actcaacata cttcatctat gaggggggtt 120

tactatcctg atgaaatttt tagatcagac actctttatt taactcagga tttatttctt 180

ccattttatt ctaatgttac agggtttcat actattaatc atacgtttgg caaccctgtc 240

atacctttta aggatggtat ttattttgct gccacagaga aatcaaatgt tgtccgtggt 300

tgggtttttg gttctaccat gaacaacaag tcacagtcgg tgattattat taacaattct 360

actaatgttg ttatacgagc atgtaacttt gaattgtgtg acaacccttt ctttgctgtt 420

tctaaaccca tgggtacaca gacacatact atgatattcg ataatgcatt taattgcact 480

ttcgagtaca tatctgatgc cttttcgctt gatgtttcag aaaagtcagg taattttaaa 540

cacttacgag agtttgtgtt taaaaataaa gatgggtttc tctatgttta taagggctat 600

caacctatag atgtagttcg tgatctacct tctggtttta acactttgaa acctattttt 660

aagttgcctc ttggtattaa cattacaaat tttagagcca ttcttacagc cttttcacct 720

gctcaagaca tttggggcac gtcagctgca gcctattttg ttggctattt aaagccaact 780

acatttatgc tcaagtatga tgaaaatggt acaatcacag atgctgttga ttgttctcaa 840

aatccacttg ctgaactcaa atgctctgtt aagagctttg agattgacaa aggaatttac 900

cagacctcta atttcagggt tgttccctca ggagatgttg tgagattccc taatattaca 960
aacttgtgtc cttttggaga ggtttttaat gctactaaat tcccttctgt ctatgcatgg 1020
gagagaaaaa aaatttctaa ttgtgttgct gattactctg tgctctacaa ctcaacattt 1080
ttttcaacct ttaagtgcta tggcgtttct gccactaagt tgaatgatct ttgcttctcc 1140
aatgtctatg cagattcttt tgtagtcaag ggagatgatg taagacaaat agcgccagga 1200
caaactggtg ttattgctga ttataattat aaattgccag atgatttcat gggttgtgtc 1260
cttgcttgga atactaggaa cattgatgct acttcaactg gtaattataa ttataaatat 1320
aggtatctta gacatggcaa gcttaggccc tttgagagag acatatctaa tgtgcctttc 1380
tcccctgatg gcaaaccttg caccccacct gctcttaatt gttattggcc attaaatgat 1440
tatggttttt acaccactac tggcattggc taccaacctt acagagttgt agtactttct 1500
tttgaacttt taaatgcacc ggccacggtt tgtggaccaa aattatccac tgaccttatt 1560
aagaaccagt gtgtcaattt taattttaat ggactcactg gtactggtgt gttaactcct 1620
tcttcaaaga gatttcaacc atttcaacaa tttggccgtg atgtttctga tttcactgat 1680
tccgttcgag atcctaaaac atctgaaata ttagacattt caccttgctc ttttgggggt 1740
gtaagtgtaa ttacacctgg aacaaatgct tcatctgaag ttgctgttct atatcaagat 1800
gttaactgca ctgatgtttc tacagcaatt catgcagatc aactcacacc agcttggcgc 1860
atatattcta ctggaaacaa tgtattccag actcaagcag gctgtcttat aggagctgag 1920
catgtcgaca cttcttatga gtgcgacatt cctattggag ctggcatttg tgctagttac 1980
catacagttt ctttattacg tagtactagc caaaaatcta ttgtggctta tactatgtct 2040
ttaggtgctg atagttcaat tgcttactct aataacacca ttgctatacc tactaacttt 2100
tcaattagca ttactacaga agtaatgcct gtttctatgg ctaaaacctc cgtagattgt 2160
aatatgtaca tctgcggaga ttctactgaa tgtgctaatt tgcttctcca atatggtagc 2220
ttttgcacac aactaaatcg tgcactctca ggtattgctg ctgaacagga tcgcaacaca 2280
cgtgaagtgt tcgctcaagt caaacaaatg tacaaaaccc caactttgaa atattttggt 2340
ggttttaatt tttcacaaat attacctgac cctctaaagc caactaagag gtcttttatt 2400
gaggacttgc tctttaataa ggtgacactc gctgatgctg gcttcatgaa gcaatatggc 2460
gaatgcctag gtgatattaa tgctagagat ctcatttgtg cgcagaagtt caatggactt 2520
acagtgttgc cacctctgct cactgatgat atgattgctg cctacactgc tgctctagtt 2580
agtggtactg ccactgctgg atggacattt ggtgctggcg ctgctcttca aatacctttt 2640
gctatgcaaa tggcatatag gttcaatggc attggagtta cccaaaatgt tctctatgag 2700

aaccaaaaac aaatcgccaa ccaatttaac aaggcgatta gtcaaattca agaatcactt 2760

acaacaacat caactgcatt gggcaagctg caagacgttg ttaaccagaa tgctcaagca 2820

ttaaacacac ttgttaaaca acttagctct aattttggtg caatttcaag tgtgctaaat 2880

gatatccttt cgcgacttga taaagtcgag gcggaggtac aaattgacag gttaattaca 2940

ggcagacttc aaagccttca aacctatgta acacaacaac taatcagggc tgctgaaatc 3000

agggcttctg ctaatcttgc tgctactaaa atgtctgagt gtgttcttgg acaatcaaaa 3060

agagttgact tttgtggaaa gggctaccac cttatgtcct tcccacaagc agccccgcat 3120

ggtgttgtct tcctacatgt cacgtatgtg ccatcccagg agaggaactt caccacagcg 3180

ccagcaattt gtcatgaagg caaagcatac ttccctcgtg aaggtgtttt tgtgtttaat 3240

ggcacttctt ggtttattac acagaggaac ttcttttctc cacaaataat tactacagac 3300

aatacatttg tctcaggaaa ttgtgatgtc gttattggca tcattaacaa cacagtttat 3360

gatcctctgc aacctgagct cgactcattc aaagaagagc tggacaagta cttcaaaaat 3420

catacatcac cagatgttga tcttggcgac atttcaggca ttaacgcttc tgtcgtcaac 3480

attcaaaaag aaattgaccg cctcaatgag gtcgctaaaa atttaaatga atcactcatt 3540

gaccttcaag aattgggaaa atatgagcaa tatattaaat ggccttggta tgtttggctc 3600

ggcttcattg ctggactaat tgccatcgtc atggttacaa tcttgctttg ttgcatgact 3660

agttgttgca gttgcctcaa gggtgcatgc tcttgtggtt cttgctgcaa gtttgatgag 3720

gatgactctg agccagttct caagggtgtc aaattacatt acacataa 3768

<210> 5

<211> 666

<212> DNA

<213> SARS-CoV

<400> 5
atggcagaca acggtactat taccgttgag gagcttaaac aactcctgga acaatggaac 60

ctagtaatag gtttcctatt cctagcctgg attatgttac tacaatttgc ctattctaat 120

cggaacaggt ttttgtacat aataaagctt gttttcctct ggctcttgtg gccagtaaca 180

cttgcttgtt ttgtgcttgc tgctgtctac agaattaatt gggtgactgg cgggattgcg 240

attgcaatgg cttgtattgt aggcttgatg tggcttagct acttcgttgc ttccttcagg 300

ctgtttgctc gtacccgctc aatgtggtca ttcaacccag aaacaaacat tcttctcaat 360

gtgcctctcc gggggacaat tgtgaccaga ccgctcatgg aaagtgaact tgtcattggt 420

gctgtgatca ttcgtggtca cttgcgaatg gccggacacc ccctagggcg ctgtgacatt 480

aaggacctgc caaaagagat cactgtggct acatcacgaa cgctttctta ttacaaatta 540

ggagcgtcgc agcgtgtagg cactgattca ggttttgctg catacaaccg ctaccgtatt 600

ggaaactata aattaaatac agaccacgcc ggtagcaacg acaatattgc tttgctagta 660

cagtaa 666

<210> 6

<211> 231

<212> DNA

<213> SARS-CoV

<400> 6
atgtactcat tcgtttcgga agaaacaggt acgttaatag ttaatagcgt acttcttttt 60

cttgctttcg tggtattctt gctagtcaca ctagccatcc ttactgcgct tcgattgtgt 120

gcgtactgct gcaatattgt taacgtgagt ttagtaaaac caacggttta cgtctactcg 180

cgtgttaaaa atctgaactc ttctgaagga gttcctgatc ttctggtcta a 231

<210> 7

<211> 22

<212> DNA

<213> Artificial Sequence

<220>

<223> oligonucleotide primer

<400> 7
tgcttccctc tgcgtagaag cc 22

<210> 8

<211> 22

<212> DNA

<213> Artificial Sequence

<220>

<223> oligonucleotide primer

<400> 8

gccaaccaac ctcgatctct tg                                                22

<210>  9

<211>  21

<212>  DNA

<213>  Artificial Sequence

<220>

<223>  oligonucleotide primer

<400>  9
tgggtccacc aaatgtaatg c                                                 21

<210>  10

<211>  21

<212>  DNA

<213>  Artificial Sequence

<220>

<223>  oligonucleotide primer

<400>  10
aagccaacca acctcgatct c                                                 21